# EUROPÄISCHE PATENTSCHRIFT

(11) **EP 2 714 013 B1**
(45) Veröffentlichungstag und Bekanntmachung des Hinweises auf die Patenterteilung: **20.02.2019**
(21) Anmeldenummer: 12737470.0
(22) Anmeldetag: 24.05.2012
(51) Int. Cl.: A61K 9/20

(54) **PULVERFÖRMIGE MISCHUNG ZUR HERSTELLUNG VON LEVETIRACETAM-HALTIGEN TABLETTEN**
POWDERY MIXTURE FOR PRODUCING TABLETS CONTAINING LEVETIRACETAM
MÉLANGE PULVÉRULENT POUR LA PRÉPARATION DE COMPRIMÉS CONTENANT DU LÉVÉTIRACÉTAM

(30) Priorität: 26.05.2011 DE 102011103270
(43) Veröffentlichungstag der Anmeldung: 09.04.2014
(73) Patentinhaber: STADA ARZNEIMITTEL AG, 61118 Bad Vilbel (DE)
(72) Erfinder: SCHÖNBORN, Jessica, 61118 Bad Vilbel (DE)
(74) Vertreter: Kernebeck, Thomas
(86) Internationale Anmeldenummer: PCT/DE2012/000542
(87) Internationale Veröffentlichungsnummer: WO 2012/159609

(56) Entgegenhaltungen:
- CN-A- 102 038 657
- S. ALI, N. LANGLEY: "Dry Granulation Simplifies Tableting Process", PHARMACEUTICAL FORMULATION & QUALITY, Bd. 12, Nr. 2, 1. Mai 2010 (2010-05-01), Seiten 26-29, XP002683248,

## Beschreibung

Die vorliegende Erfindung betrifft eine pulverförmige Mischung zur Herstellung von Levetiracetam-haltigen Tabletten durch Direkttablettieren der Mischung, wobei die Mischung mindestens 60 Gew.-% Levetiracetam sowie ein Trockenbindemittel umfasst.

Levetiracetam ist der internationale Freiname (INN (International Nonproprietary Name)-Name) für (S)-2-(2-Oxopyrrolidin-1-yl)-butylamid. Levetiracetam weist die folgende Strukturformel auf:

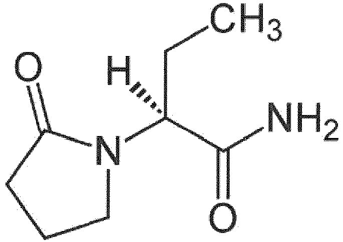

Levetiracetam ist ein Arzneistoff aus der Gruppe der Antieleptika und ist zur Behandlung von Epilepsie zugelassen. Der Wirkungsmechanismus von Levetiracetam ist unbekannt (vgl. Mutschler Arzneimittelwirkungen, Lehrbuch der Pharmakologie und Toxikologie, 9. Auflage, Wissenschaftliche Verlagsgesellschaft mbH Stuttgart, 2008, S. 318; ISBN 978-3-8047-1952-1).

Um eine angemessene pharmazeutische Wirkung zu gewährleisten, muss Levetiracetam in verhältnismäßig hohen Dosen verabreicht werden. Zur Vermeidung einer gleichzeitigen Einnahme mehrerer Levetiracetam-Darreichungsformen und einer damit einhergehenden erhöhten Patientenakzeptanz sind im Stand der Technik Filmtabletten, wie z.B. Keppra®, bekannt, die einen relativ hohen Wirkstoffgehalt aufweisen. Diese Darreichungsform erlaubt die Verabreichung der zu applizierenden Dosis durch Einnahme einer einzigen Filmtablette.

Die CN 102038657 A offenbart Formulierungen zur Direkttablettierung von Levetiracetam mit Copovidon als Trockenbindemittel.

Levetiracetam besitzt jedoch relativ schlechte Verpressungseigenschaften, weshalb Tabletten mit vergleichsweise hohen Levetiracetam-Gehalten verhältnismäßig schlechte mechanische Eigenschaften aufweisen. Insbesondere weisen entsprechende Tabletten hohe Friabilitäts-Werte auf, weshalb die Tabletten beispielsweise nur schwer befilmbar sind bzw. die aufgetragenen Filme nicht ausreichend auf der Oberfläche der Tablette anhaften und sich entsprechend leicht ablösen.

Aufgabe der vorliegenden Erfindung ist es daher, eine pulverförmige Mischung mit einem relativ hohen Gehalt an Levetiracetam bereitzustellen, mittels welcher Tabletten herstellbar sind, die sich durch verhältnismäßig niedrige Werte der Friabilität auszeichnen und entsprechend gut befilmbar sind.

Diese Aufgabe wird erfindungsgemäß gelöst durch eine pulverförmige Mischung zur Herstellung von Levetiracetam-haltigen Tabletten durch Direkttablettieren der Mischung, wobei die Mischung mindestens 60 Gew.-% Levetiracetam sowie ein Trockenbindemittel umfasst und wobei das Trockenbindemittel einen D10-Wert von 1 *µ*m bis 15 *µ*m, einen D50-Wert von 10 *µ*m bis 50 *µ*m und einen D90-Wert von 30 *µ*m bis 120 *µ*m aufweist, wobei das Trockenbindemittel ein Vinylpyrrolidon-Vinylacetat-Copolymer ist

Überraschenderweise wurde festgestellt, dass mittels der erfindungsgemäßen Mischung Tabletten erhältlich sind, die vergleichsweise geringe Werte der Friabilität aufweisen. So können mittels der erfindungsgemäßen Mischung Friabilitäts-Werte von bis zu kleiner/gleich 0,3 % erzielt werden, wobei die erreichbaren Werte insbesondere abhängig sind von der Natur des Trockenbindemittels, seinem Anteil in der erfindungsgemäßen Mischung sowie vom Verpressungsdruck. Die Friabilität ist gemäß dem Europäischen Arzneibuch (Ph.Eur.), 6. Ausgabe, 6. Nachtrag, S. 6820-6821, Kapitel 2.9.7 "Friabilität von nicht überzogenen Tabletten" zu bestimmen.

Die erfindungsgemäße pulverförmige Mischung ist zur Herstellung von Levetiracetam-haltigen Tabletten durch Direkttablettieren der Mischung geeignet. Unter dem Begriff "Direkttablettieren" wird dabei, wie im Stand der Technik bekannt, das Verpressen eines Pulvers ohne weitere Vorbehandlung desselben - wie etwa ein Granulieren - unter Erhalt einer Tablette verstanden.

Die erfindungsgemäße Mischung ist darüber hinaus aber auch zur Weiterverarbeitung mittels anderer Verfahren und/oder zu anderen Formkörpern als Tabletten, wie etwa Briketts, geeignet. So kann die erfindungsgemäße Mischung beispielsweise trockengranuliert und das resultierende Trockengranulat gegebenenfalls nach Zugabe eines oder mehrerer pharmazeutischer Hilfsstoffe zu Tabletten verpresst werden.

Das in der erfindungsgemäßen Mischung enthaltene Trockenbindemittel weist einen D10-Wert von 1 *µ*m bis 15 *µ*m, einen D50-Wert von 10 *µ*m bis 50 *µ*m und einen D90-Wert von 30 *µ*m bis 120 *µ*m auf. Unter D10-Wert wird im Rahmen der vorliegenden Erfindung in diesem Zusammenhang die Partikelgröße verstanden, bei der 10 % der Partikel bezogen auf das Volumen kleiner als der D10-Wert sind und 90 % der Partikel bezogen auf das Volumen größer sind als der D10-Wert. In Analogie dazu wird unter D50-Wert die Partikelgröße verstanden, bei der 50 % der Partikel bezogen auf das Volumen kleiner als der D50-Wert sind und 50 % der Partikel bezogen auf das Volumen größer sind als der D50-Wert. In Analogie dazu wird unter D90-Wert die Partikelgröße verstanden, bei der 90 % der Partikel bezogen auf das Volumen kleiner als der D90-Wert sind und 10 % der Partikel bezogen auf das Volumen größer sind als der D90-Wert.

Die Bestimmung des D10-, D50- und D90-Wertes erfolgt mittels Laserbeugung unter Einsatz eines Gerätes mit der Bezeichnung "Mastersizer 2000" der Firma "Malvern Instruments" gemäß dem Europäischen Arzneibuch (Ph. Eur.) 6. Ausgabe, 6. Nachtrag, Kapitel 2.9.31 "Bestimmung der Partikelgröße durch Laserdiffraktometrie", Seiten 6825-6830. Die Einstellungen sind dabei wie folgt: Dispergiersystem (dispersing system) Scirocco 2000; Messbereich (result range) 0,02 bis 2000 *µ*m; Auswertung (reporting) Mie; Berechnungsmodel (calculation model) Mie; Berechnungsempfindlichkeit (calculation sensitivity) universell (general purpose); Messzeit (measurement time) 5 s; Hintergrundzeit (background time) 5 s; Messaufnahmen (measurement snaps) 5000; Hintergrundaufnahmen (background snaps) 5000; Dispergiermethode (dispersing method) Trockenmessung, das Pulver wird in Luft dispergiert; Einsatz (tray) universell (general purpose); Probennehmereinstellungen (sampler settings) Vibrationszuführrate (vibration feed rate) 60 %, Luftdruck zur Dispergierung (dispersive air pressure) 2,5 bar; Trübungsgrenzen (obscuration limits) Untergrenze 2, Obergrenze 10; Anzahl der Messungen 2.

Entsprechend einer bevorzugten Ausführungsform der erfindungsgemäßen Mischung ist es vorgesehen, dass das Trockenbindemittel einen D10-Wert von 1 *µ*m bis 5 *µ*m, einen D50-Wert von 10 *µ*m bis 15 *µ*m und einen D90-Wert von 30 *µ*m bis 40 *µ*m aufweist. Es konnte festgestellt werden, dass eine erfindungsgemäße Mischung mit einem Trockenbindemittel mit einem D10-, D50- und D90-Wert in den genannten Bereichen zu Tabletten verarbeitet werden kann, die sich durch einen besonders niedrigen Abrieb (Friabilität) auszeichnen.

Gemäß einer weiter bevorzugten Ausführungsform der erfindungsgemäßen Mischung beträgt der Anteil der Mischung an Levetiracetam 60 Gew.-% bis 95 Gew.-% und mehr bevorzugt 70 Gew.-% bis 90 Gew.-%. Die erfindungsgemäße Mischung kann Levetiracetam in den genannten, relativ hohen Gehalten enthalten und kann trotzdem zu Tabletten mit einer niedrigen Friabilität direktverpresst werden.

Entsprechend einer besonders bevorzugten Ausführungsform der erfindungsgemäßen Mischung ist es vorgesehen, dass der Anteil der Mischung an Levetiracetam 80 Gew.-% bis 90 Gew.-% beträgt.

Es konnte festgestellt werden, dass mittels der erfindungsgemäßen pulverförmigen Mischung insbesondere dann Tabletten mit einer niedrigen Friabilität durch Direktverpressen erhältlich sind, wenn das Levetiracetam einen D90-Wert von kleiner als 500 *µ*m aufweist. Entsprechend ist es gemäß einer weiter bevorzugten Ausführungsform der erfindungsgemäßen Mischung vorgesehen, dass das Levetiracetam einen D90-Wert von kleiner als 500 *µ*m aufweist, vorzugsweise einen D90-Wert von 50 *µ*m bis 490 *µ*m. Unter D90-Wert wird im Rahmen der vorliegenden Erfindung in diesem Zusammenhang die Partikelgröße verstanden bei der 90 % der Partikel bezogen auf das Volumen kleiner als der D90-Wert sind und 10 % der Partikel bezogen auf das Volumen größer sind als der D90-Wert.

Die Bestimmung des D90-Wertes des Levetiracetams erfolgt analog wie fünf Absätze vorstehend unter "Die Bestimmung des D10-, D50- und D90-Wertes" ausgeführt.

In der erfindungsgemäßen Mischung ist ein Trockenbindemittel mit einem D10-Wert von 1 *µ*m bis 15 *µ*m, einem D50-Wert von 10 *µ*m bis 50 *µ*m und einem D90-Wert von 30 *µ*m bis 120 *µ*m enthalten. Als Trockenbindemittel kann dabei im Prinzip jedes dem Fachmann aus dem Stand der Technik bekannte pharmazeutische Trockenbindemittel eingesetzt sein, dass der genannten Partikelgrößenverteilung genügt. Erfindungsgemäß ist das Trockenbindemittel ein Vinylpyrrolidon-Vinylacetat-Copolymer. Vinylpyrrolidon-Vinylacetat-Copolymer-Trockenbindemittel können in der erfindungsgemäßen Mischung in verhältnismäßig geringer Menge enthalten sein und bewirken dennoch eine hohe Abriebfestigkeit von der Mischung durch Direktverpressen hergestellten Tabletten.

Ein erfindungsgemäß besonders bevorzugtes Vinylpyrrolidon-Vinylacetat-Copolymer-Trockenbindemittel wird von der BASF AG, Ludwigshafen, Deutschland unter der Bezeichnung Kollidon® VA 64 angeboten. Dieses Trockenbindemittel weist ein Vinylpyrrolidon/Vinylacetat-Massenverhältnis von 6:4 auf.

Gemäß einer weiter bevorzugten Ausführungsform der erfindungsgemäßen Mischung ist es vorgesehen, dass der Anteil der Mischung an dem Trockenbindemittel 0,5 Gew.-% bis 10 Gew.-% oder 2,0 Gew.-% bis 10 Gew.-% beträgt, vorzugsweise 3 Gew.-% bis 8 Gew.-%. Der Anteil der erfindungsgemäßen Mischung an Trockenbindemittel kann vergleichsweise gering gewählt sein, ohne dadurch die Friabilität der aus der Mischung herzustellenden Tabletten signifikant zu erhöhen. Dadurch wird ein maximaler Wirkstoffgehalt in der Mischung und damit in der resultierenden Tablette gewährleistet.

Entsprechend einer weiter bevorzugten Ausführungsform der erfindungsgemäßen Mischung ist es vorgesehen, dass die Mischung ferner einen oder mehrere pharmazeutische Hilfsstoffe umfasst. Erfindungsgemäß bevorzugte Hilfsstoffe sind ausgewählt aus der Gruppe bestehend aus einem Füllmittel, Füllmittelgemisch, Fließregulierungsmittel, Fließregulierungsmittelgemisch, Schmiermittel, Schmiermittelgemisch, Formentrennmittel und einem Formentrennmittelgemisch.

Der Anteil der erfindungsgemäßen Mischung an pharmazeutischem/n Hilfsstoff/en beträgt erfindungsgemäß bevorzugt 0,5 Gew.-% bis 30 Gew.-% und weiter bevorzugt 5 Gew.-% bis 10 Gew.-%.

Die vorliegende Erfindung betrifft ferner eine Levetiracetam-haltige Tablette erhältlich durch Direkttablettieren einer erfindungsgemäßen pulverförmigen Mischung.

Die erfindungsgemäße Levetiracetam-haltige Tablette ist durch Direkttablettieren einer erfindungsgemäßen pulverförmigen Mischung erhältlich. Entsprechend umfasst die Tablette die erfindungsgemäße Mischung in kompaktierter Form.

Die erfindungsgemäße Tablette weist erfindungsgemäß bevorzugt eine Friabilität von kleiner als 1 % auf, mehr bevorzugt eine Friabilität von kleiner als 0,8 %, weiter bevorzugt eine Friabilität von kleiner als 0,5 % und besonders bevorzugt eine Friabilität von kleiner als 0,3 %.

Die Friabilitäts-Werte der erfindungsgemäßen Tablette sind insbesondere abhängig von der Natur des Trockenbindemittels, seinem Anteil in der erfindungsgemäßen Mischung sowie vom Verpressungsdruck bei der Herstellung der Tablette. Die Friabilität ist gemäß dem Europäischen Arzneibuch (Ph.Eur.), 6. Ausgabe, 6. Nachtrag, S. 6820-6821, Kapitel 2.9.7 "Friabilität von nicht überzogenen Tabletten" zu bestimmen.

Die erfindungsgemäße Tablette kann, wenn gewünscht, unter Erhalt einer Filmtablette befilmt werden

Die vorliegende Offenbarung betrifft ferner die Verwendung eines Trockenbindemittels mit einem D10-Wert von 1 *µ*m bis 15 *µ*m, einem D50-Wert von 10 *µ*m bis 50 *µ*m und einem D90-Wert von 30 *µ*m bis 120 *µ*m als Trockenbindemittel bei der Herstellung von Levetiracetam-haltigen Presslingen, vorzugsweise Tabletten oder Briketts.

Entsprechend einer bevorzugten Ausführungsform der offenbarten Verwendung ist es vorgesehen, dass das Trockenbindemittel einen D10-Wert von 1 *µ*m bis 5 *µ*m, einen D50-Wert von 10 *µ*m bis 15 *µ*m und einen D90-Wert von 30 *µ*m bis 40 *µ*m aufweist.

Gemäß einer weiter bevorzugten Ausführungsform der offenbarten Verwendung ist das Trockenbindemittel ein Vinylpyrrolidon-Vinylacetat-Copolymer. Ein besonders geeignetes Vinylpyrrolidon-Vinylacetat-Copolymer-Trockenbindemittel wird von der BASF AG, Ludwigshafen, Deutschland unter der Bezeichnung Kollidon® VA 64 angeboten. Dieses Trockenbindemittel weist ein Vinylpyrrolidon/Vinylacetat-Massenverhältnis von 6:4 auf.

Die vorliegende Erfindung betrifft ferner ein Verfahren zur Herstellung einer Levetiracetam-haltigen Tablette, umfassend den Schritt
- Tablettieren, vorzugsweise Direkttablettieren, einer erfindungsgemäßen pulverförmigen Mischung.

Die nachfolgenden Ausführungsbeispiele dienen der Erläuterung der Erfindung.

### Ausführungsbeispiel 1:

Es wurde ein Gemisch als erfindungsgemäße pulverförmige Mischung enthaltend
- 88,0 Gew.-% Levetiracetam mit einem D90-Wert von 440 *µ*m;
- 5,0 Gew.-% Kollidon® VA 64 (BASF AG, Ludwigshafen, Deutschland) als Trockenbindemittel mit einem D10-Wert von 4 *µ*m, einem D50-Wert von 13 *µ*m und einem D90-Wert von 34 *µ*m;
- 5,5 Gew.-% mikrokristalline Cellulose;
- 1,0 Gew.-% Siliciumdioxid; und
- 0,5 Gew.-% Magnesiumstearat
hergestellt. Die Herstellung des Gemisches erfolgte durch Zusammengeben der genannten Substanzen und Vermischen derselben bis zur Homogenität in einem Mischer.

### Ausführungsbeispiel 2:

Das Gemisch gemäß Ausführungsbeispiel 1 wurde mittels einer konventionellen Tablettenpresse zu Tablettenkernen als Presslinge verpresst. Die so erhaltenen Tablettenkerne weisen eine Friabilität von 0,03 % auf. Dabei wird die Friabilität gemäß Ph.Eur., 6. Ausgabe, 6. Nachtrag, S. 6820-6821, Kapitel 2.9.7 "Friabilität von nicht überzogenen Tabletten" bestimmt.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Suspendieren des kommerziell erhältlichen Befilmungssystems Opadry® blau in Wasser. Die resultierenden Filmtabletten weisen die in der Tabelle 1 angegebene Zusammensetzung auf:

**Tabelle 1:**

| **Substanz** | **Menge pro Tablette [mg]** | **Funktion der Substanz** |
|---|---|---|
| **Tablettenkern** | | |
| Levetiracetam | 250,000 | Wirkstoff |
| Kollidon® VA 64 | 14,225 | Trockenbindemittel |
| mikrokristalline Cellulose | 15,775 | Füllmittel |
| hochdisperses Siliciumdioxid | 3,000 | Fließregulierungsmittel |
| Magnesiumstearat | 1,500 | Schmiermittel |

| **Film** | | |
|---|---|---|
| Opadry® blau (enthält Hypromellose, Polyethylenglycol (Macrogol®), Talkum, Titandioxid, Farbstoff) | 9,500 | vollständiges Befilmungssystem |
| **Gesamtgewicht der Filmtablette** | 294,000 | |

Der Film haftet auf den Tablettenkernen sehr gut.

### Ausführungsbeispiel 3:

Das Gemisch gemäß Ausführungsbeispiel 1 wurde analog Ausführungsbeispiel 2 zu Tablettenkernen und diese zu Filmtabletten weiterverarbeitet. Die erhaltenen Tablettenkerne weisen eine Friabilität von 0,07 % auf, die gemäß der in Ausführungsbeispiel 2 genannten Vorschrift bestimmt wurde.

Die resultierenden Filmtabletten weisen die in der Tabelle 2 angegebene Zusammensetzung auf:

**Tabelle 2:**

| **Substanz** | **Menge pro Tablette [mg]** | **Funktion der Substanz** |
|---|---|---|
| **Tablettenkern** | | |
| Levetiracetam | 500,00 | Wirkstoff |
| Kollidon® VA 64 | 28,45 | Trockenbindemittel |
| mikrokristalline Cellulose | 31,55 | Füllmittel |
| hochdisperses Siliciumdioxid | 6,00 | Fließregulierungsmittel |
| Magnesiumstearat | 3,00 | Schmiermittel |

| **Film** | | |
|---|---|---|
| Opadry® gelb (enthält Hypromellose, Polyethylenglycol (Macrogol®), Talkum, Titandioxid, Farbstoff) | 19,00 | vollständiges Befilmungssystem |
| **Gesamtgewicht der Filmtablette** | 588,00 | |

Der Film haftet auf den Tablettenkernen sehr gut.

### Ausführungsbeispiel 4:

Das Gemisch gemäß Ausführungsbeispiel 1 wurde analog Ausführungsbeispiel 2 zu Tablettenkernen und diese zu Filmtabletten weiterverarbeitet. Die erhaltenen Tablettenkerne weisen eine Friabilität von 0,06 % auf, die gemäß der in Ausführungsbeispiel 2 genannten Vorschrift bestimmt wurde.

Die resultierenden Filmtabletten weisen die in der Tabelle 3 angegebene Zusammensetzung auf:

**Tabelle 3:**

| **Substanz** | **Menge pro Tablette [mg]** | **Funktion der Substanz** |
|---|---|---|
| **Tablettenkern** | | |
| Levetiracetam | 750,000 | Wirkstoff |
| Kollidon® VA 64 | 42,675 | Trockenbindemittel |
| mikrokristalline Cellulose | 47,325 | Füllmittel |
| hochdisperses Siliciumdioxid | 9,000 | Fließregulierungsmittel |
| Magnesiumstearat | 4,500 | Schmiermittel |

| **Film** | | |
|---|---|---|
| Opadry® orange (enthält Hypromellose, Polyethylenglycol (Macrogol®), Talkum, Titandioxid, Farbstoff) | 28,500 | vollständiges Befilmungssystem |
| **Gesamtgewicht der Filmtablette** | 882,000 | |

Der Film haftet auf den Tablettenkernen sehr gut.

### Ausführungsbeispiel 5:

Das Gemisch gemäß Ausführungsbeispiel 1 wurde analog Ausführungsbeispiel 2 zu Tablettenkernen und diese zu Filmtabletten weiterverarbeitet. Die erhaltenen Tablettenkerne weisen eine Friabilität von 0,23 % auf, die gemäß der in Ausführungsbeispiel 2 genannten Vorschrift bestimmt wurde.

Die resultierenden Filmtabletten weisen die in der Tabelle 4 angegebene Zusammensetzung auf:

**Tabelle 4:**

| **Substanz** | **Menge pro Tablette [mg]** | **Funktion der Substanz** |
|---|---|---|
| **Tablettenkern** | | |
| Levetiracetam | 1000,00 | Wirkstoff |
| Kollidon® VA 64 | 56,90 | Trockenbindemittel |
| mikrokristalline Cellulose | 63,10 | Füllmittel |
| hochdisperses Siliciumdioxid | 12,00 | Fließregulierungsmittel |
| Magnesiumstearat | 6,00 | Schmiermittel |

| **Film** | | |
|---|---|---|
| Opadry® gelb (enthält Hypromellose, Polyethylenglycol (Macrogol®), Talkum, Titandioxid, Farbstoff) | 38,00 | vollständiges Befilmungssystem |
| **Gesamtgewicht der Filmtablette** | 1176,00 | |

Der Film haftet auf den Tablettenkernen sehr gut.

### Vergleichsbeispiel 1:

Es wurde ein Gemisch als pulverförmige Mischung enthaltend
- 88,0 Gew.-% Levetiracetam mit einem D90-Wert von 440 *µ*m;
- 5,0 Gew.-% Kollidon® 25 (BASF AG, Ludwigshafen, Deutschland) als Trockenbindemittel mit einem D10-Wert von 19 *µ*m, einem D50-Wert von 54 *µ*m und einem D90-Wert von 106 *µ*m;
- 5,5 Gew.-% mikrokristalline Cellulose;
- 1,0 Gew.-% Siliciumdioxid; und
- 0,5 Gew.-% Magnesiumstearat
hergestellt. Die Herstellung des Gemisches erfolgte durch Zusammengeben der genannten Substanzen und Vermischen derselben bis zur Homogenität in einem Mischer.

### Vergleichsbeispiel 2:

Das Gemisch gemäß Vergleichsbeispiel 1 wurde mittels einer konventionellen Tablettenpresse zu Tablettenkernen als Presslinge verpresst. Die so erhaltenen Tablettenkerne weisen eine Friabilität von 0,5 % auf. Dabei wird die Friabilität gemäß Ph.Eur., 6. Ausgabe, 6. Nachtrag, S. 6820-6821, Kapitel 2.9.7 "Friabilität von nicht überzogenen Tabletten" bestimmt.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Suspendieren des kommerziell erhältlichen Befilmungssystems Opadry® blau in Wasser. Die resultierenden Filmtabletten weisen die in der Tabelle 5 angegebene Zusammensetzung auf:

**Tabelle 5:**

| **Substanz** | **Menge pro Tablette [mg]** | **Funktion der Substanz** |
|---|---|---|
| **Tablettenkern** | | |
| Levetiracetam | 250,000 | Wirkstoff |
| Kollidon® 25 | 14,225 | Trockenbindemittel |
| mikrokristalline Cellulose | 15,775 | Füllmittel |
| hochdisperses Siliciumdioxid | 3,000 | Fließregulierungsmittel |
| Magnesiumstearat | 1,500 | Schmiermittel |

| **Film** | | |
|---|---|---|
| Opadry® blau (enthält Hypromellose, Polyethylenglycol (Macrogol®), Talkum, Titandioxid, Farbstoff) | 9,500 | vollständiges Befilmungssystem |
| **Gesamtgewicht der Filmtablette** | 294,000 | |

Der Film haftet auf den Tablettenkernen verhältnismäßig schlecht.

### Vergleichsbeispiel 3:

Das Gemisch gemäß Vergleichsbeispiel 1 wurde analog Vergleichsbeispiel 2 zu Tablettenkernen und diese zu Filmtabletten weiterverarbeitet. Die erhaltenen Tablettenkerne weisen eine Friabilität von 0,7 % auf, die gemäß der in Vergleichsbeispiel 2 genannten Vorschrift bestimmt wurde.

Die resultierenden Filmtabletten weisen die in der Tabelle 6 angegebene Zusammensetzung auf:

**Tabelle 6:**

| **Substanz** | **Menge pro Tablette [mg]** | **Funktion der Substanz** |
|---|---|---|
| **Tablettenkern** | | |
| Levetiracetam | 500,00 | Wirkstoff |
| Kollidon® 25 | 28,45 | Trockenbindemittel |
| mikrokristalline Cellulose | 31,55 | Füllmittel |
| hochdisperses Siliciumdioxid | 6,00 | Fließregulierungsmittel |
| Magnesiumstearat | 3,00 | Schmiermittel |

| **Film** | | |
|---|---|---|
| Opadry® gelb (enthält Hypromellose, Polyethylenglycol (Macrogol®), Talkum, Titandioxid, Farbstoff) | 19,00 | vollständiges Befilmungssystem |
| **Gesamtgewicht der Filmtablette** | 588,00 | |

Der Film haftet auf den Tablettenkernen verhältnismäßig schlecht.

### Vergleichsbeispiel 4:

Das Gemisch gemäß Vergleichsbeispiel 1 wurde analog Ausführungsbeispiel 2 zu Tablettenkernen und diese zu Filmtabletten weiterverarbeitet. Die erhaltenen Tablettenkerne weisen eine Friabilität von 0,8 % auf, die gemäß der in Ausführungsbeispiel 2 genannten Vorschrift bestimmt wurde.

Die resultierenden Filmtabletten weisen die in der Tabelle 7 angegebene Zusammensetzung auf:

**Tabelle 7:**

| **Substanz** | **Menge pro Tablette [mg]** | **Funktion der Substanz** |
|---|---|---|
| **Tablettenkern** | | |
| Levetiracetam | 750,000 | Wirkstoff |
| Kollidon® 25 | 42,675 | Trockenbindemittel |
| mikrokristalline Cellulose | 47,325 | Füllmittel |
| hochdisperses Siliciumdioxid | 9,000 | Fließregulierungsmittel |
| Magnesiumstearat | 4,500 | Schmiermittel |

| **Film** | | |
|---|---|---|
| Opadry® orange (enthält Hypromellose, Polyethylenglycol (Macrogol®), Talkum, Titandioxid, Farbstoff) | 28,500 | vollständiges Befilmungssystem |
| **Gesamtgewicht der Filmtablette** | 882,000 | |

Der Film haftet auf den Tablettenkernen verhältnismäßig schlecht.

### Vergleichsbeispiel 5:

Das Gemisch gemäß Vergleichsbeispiel 1 wurde analog Vergleichsbeispiel 2 zu Tablettenkernen und diese zu Filmtabletten weiterverarbeitet. Die erhaltenen Tablettenkerne weisen eine Friabilität von 1,1 % auf, die gemäß der in Ausführungsbeispiel 2 genannten Vorschrift bestimmt wurde.

Die resultierenden Filmtabletten weisen die in der Tabelle 8 angegebene Zusammensetzung auf:

**Tabelle 8:**

| **Substanz** | **Menge pro Tablette [mg]** | **Funktion der Substanz** |
|---|---|---|
| **Tablettenkern** | | |
| Levetiracetam | 1000,00 | Wirkstoff |
| Kollidon® 25 | 56,90 | Trockenbindemittel |
| mikrokristalline Cellulose | 63,10 | Füllmittel |
| hochdisperses Siliciumdioxid | 12,00 | Fließregulierungsmittel |
| Magnesiumstearat | 6,00 | Schmiermittel |

| **Film** | | |
|---|---|---|
| Opadry® gelb (enthält Hypromellose, Polyethylenglycol (Macrogol®), Talkum, Titandioxid, Farbstoff) | 38,00 | vollständiges Befilmungssystem |
| **Gesamtgewicht der Filmtablette** | 1176,00 | |

Der Film haftet auf den Tablettenkernen verhältnismäßig schlecht.

## Patentansprüche

1. Pulverförmige Mischung zur Herstellung von Levetiracetam-haltigen Tabletten durch Direkttablettieren der Mischung, wobei die Mischung mindestens 60 Gew.-% Levetiracetam sowie ein Trockenbindemittel umfasst, **dadurch gekennzeichnet, dass** das Trockenbindemittel einen D10-Wert von 1 *µ*m bis 15 *µ*m, einen D50-Wert von 10 *µ*m bis 50 *µ*m und einen D90-Wert von 30 *µ*m bis 120 *µ*m aufweist, wobei das Trockenbindemittel ein Vinylpyrrolidon-Vinylacetat-Copolymer ist und wobei die D-Werte gemäß dem Europäischen Arzneibuch 6. Ausgabe, 6. Nachtrag, Kapitel 2.9.31, Seiten 6825-6830 (mittels eines Gerätes mit der Bezeichnung Mastersizer 2000) bestimmt werden.

2. Mischung nach Anspruch 1, **dadurch gekennzeichnet, dass** das Trockenbindemittel einen D10-Wert von 1 *µ*m bis 5 *µ*m, einen D50-Wert von 10 *µ*m bis 15 *µ*m und einen D90-Wert von 30 *µ*m bis 40 *µ*m aufweist.

3. Mischung nach einem der voranstehenden Ansprüche, **dadurch gekennzeichnet, dass** der Anteil der Mischung an Levetiracetam 80 Gew.-% bis 90 Gew.-% beträgt.

4. Mischung nach einem der voranstehenden Ansprüche, **dadurch gekennzeichnet, dass** das Levetiracetam einen D90-Wert von kleiner als 500 *µ*m aufweist, wobei der D90-Wert gemäß dem Europäischen Arzneibuch 6. Ausgabe, 6. Nachtrag, Kapitel 2.9.31, Seiten 6825-6830 (mittels eines Gerätes mit der Bezeichnung Mastersizer 2000) bestimmt wird.

5. Mischung nach einem der voranstehenden Ansprüche, **dadurch gekennzeichnet, dass** das Levetiracetam einen D90-Wert von 50 *µ*m bis 490 *µ*m aufweist, wobei der D90-Wert gemäß dem Europäischen Arzneibuch 6. Ausgabe, 6. Nachtrag, Kapitel 2.9.31, Seiten 6825-6830 (mittels eines Gerätes mit der Bezeichnung Mastersizer 2000) bestimmt wird.

6. Mischung nach einem der voranstehenden Ansprüche, **dadurch gekennzeichnet, dass** der Anteil der Mischung an dem Trockenbindemittel 2 Gew.-% bis 10 Gew.-% beträgt, vorzugsweise 3 Gew.-% bis 8 Gew.-%.

7. Levetiracetam-haltige Tablette erhältlich durch Direkttablettieren einer pulverförmigen Mischung nach einem der Ansprüche 1 bis 6.

8. Verfahren zur Herstellung einer Levetiracetam-haltigen Tablette, umfassend den Schritt
- Tablettieren einer pulverförmigen Mischung nach einem der Ansprüche 1 bis 6.

## Claims

1. Pulverulent mixture for preparing tablets containing levetiracetam by direct compression of the mixture, wherein the mixture comprises at least 60 wt% levetiracetam and also a dry binder, **characterized in that** the dry binder has a D10 value of 1 µm to 15 µm, a D50 value of 10 µm to 50 µm and a D90 value of 30 µm to 120 µm, wherein the dry binder is a vinyl pyrrolidone-vinyl acetate copolymer and wherein the D values are determined according to the European Pharmacopoeia, 6th edition, supplement 6, chapter 2.9.31, pages 6825-6830 (by means of an apparatus with the designation Mastersizer 2000).

2. Mixture according to Claim 1, **characterized in that** the dry binder has a D10 value of 1 µm to 5 µm, a D50 value of 10 µm to 15 µm and a D90 value of 30 µm to 40 µm.

3. Mixture according to either of the preceding claims, **characterized in that** the proportion of levetiracetam in the mixture is 80 wt% to 90 wt%.

4. Mixture according to one of the preceding claims, **characterized in that** the levetiracetam has a D90 value of less than 500 µm, wherein the D90 value is determined according to the European Pharmacopoeia, 6th edition, supplement 6, chapter 2.9.31, pages 6825-6830 (by means of an apparatus with the designation Mastersizer 2000).

5. Mixture according to one of the preceding claims, **characterized in that** the levetiracetam has a D90 value of 50 µm to 490 µm, wherein the D90 value is determined according to the European Pharmacopoeia, 6th edition, supplement 6, chapter 2.9.31, pages 6825-6830 (by means of an apparatus with the designation Mastersizer 2000).

6. Mixture according to one of the preceding claims, **characterized in that** the proportion of dry binder in the mixture is 2 wt% to 10 wt%, preferably 3 wt% to 8 wt%.

7. Tablets containing levetiracetam, obtainable by direct compression of a pulverulent mixture according to one of Claims 1 to 6.

8. Method for preparing a tablet containing levetiracetam, comprising the step of:
- compression of a pulverulent mixture according to one of Claims 1 to 6.

## Revendications

1. Mélange pulvérulent pour la préparation de comprimés contenant du lévétiracétam par pastillage direct du mélange, dans lequel le mélange comprend au moins 60 % en poids de lévétiracétam ainsi qu'un liant sec, **caractérisé en ce que** le liant sec présente une valeur D10 de 1 µm à 15 µm, une valeur D50 de 10 µm à 50 µm et une valeur D90 de 30 µm à 120 µm, le liant sec étant un copolymère de vinylpyrrolidone - acétate de vinyle et les valeurs D étant déterminées selon la pharmacopée européenne 6^{e} édition, 6^{e} addendum, chapitre 2.9.31, pages 6825 à 6830 (au moyen d'un appareil portant la désignation Mastersizer 2000).

2. Mélange selon la revendication 1, **caractérisé en ce que** le liant sec présente une valeur D10 de 1 µm à 5 µm, une valeur D50 de 10 µm à 15 µm et une valeur D90 de 30 µm à 40 µm.

3. Mélange selon l'une des revendications précédentes, **caractérisé en ce que** la teneur du mélange en lévétiracétam est de 80 % en poids à 90 % en poids.

4. Mélange selon l'une des revendications précédentes, **caractérisé en ce que** le lévétiracétam présente une valeur D90 inférieure à 500 µm, la valeur D90 étant déterminée selon la pharmacopée européenne 6^{e} édition, 6^{e} addendum, chapitre 2.9.31, pages 6825 à 6830 (au moyen d'un appareil portant la désignation Mastersizer 2000).

5. Mélange selon l'une des revendications précédentes, **caractérisé en ce que** le lévétiracétam présente une valeur D90 de 50 µm à 490 µm, la valeur D90 étant déterminée selon la pharmacopée européenne 6^{e} édition, 6^{e} addendum, chapitre 2.9.31, pages 6825 à 6830 (au moyen d'un appareil portant la désignation Mastersizer 2000).

6. Mélange selon l'une des revendications précédentes, **caractérisé en ce que** la teneur du mélange en liant sec est de 2 % en poids à 10 % en poids, de préférence de 3 % en poids à 8 % en poids.

7. Comprimé contenant du lévétiracétam qui peut être obtenu par pastillage direct d'un mélange pulvérulent selon l'une des revendications 1 à 6.

8. Procédé de préparation d'un comprimé contenant du lévétiracétam, comprenant l'étape de
- pastillage d'un mélange pulvérulent selon l'une des revendications 1 à 6.
